# EUROPEAN PATENT SPECIFICATION

(11) **EP 3 656 344 B1**
(45) Date of publication and mention of the grant of the patent: **21.07.2021**
(21) Application number: 20150143.4
(22) Date of filing: 04.11.2016
(51) Int. Cl.: A61C 7/08, A61B 5/01, A61B 5/022, A61B 5/024, A61B 5/08, A61B 5/145, A61B 5/00

(54) **TOOTH WEARABLE DEVICE**
ZAHN-TRAGEGERÄT
DISPOSITIF A PORTER SUR LES DENTS

(43) Date of publication of application: 27.05.2020
(62) Divisional of application: 16197274.0
(73) Proprietor: Lee, Jin Kyun, Seoul 07020 (KR)
(72) Inventor: Lee, Jin Kyun, Seoul 07020 (KR)
(74) Representative: Berggren Oy

(56) References cited:
- EP-A1- 2 789 294
- US-A1- 2006 166 157
- US-A1- 2013 109 932

## Description

### TECHNICAL FIELD

The invention relates to a tooth-attach wearable device to detect whether the device is worn by the user and how long it is worn.

### RELATED ART

In general, types of an orthodontic device include a metal orthodontic device, a ceramic orthodontic device, a modified tandem appliance (MTA) orthodontic device, a clippy-C orthodontic device, a Damon orthodontic device, and the like.

Currently, many patients select a transparent orthodontic device among such various orthodontic devices. As for the greatest advantage, the transparent orthodontic device has an aesthetic property. That is, a patient may not appear to wear the orthodontic device. In many cases, an orthodontic treatment may be performed without tooth extraction. The patient may take out or insert the orthodontic device in person and may experience a relatively less pain compared to other wire orthodontic devices during an orthodontic treatment process.

However, since the patient may take out or insert the orthodontic device in person, the patient may have a degraded effect and experience an increased treatment term if the patient does not wear the orthodontic device in an appropriate manner.

Document US 2006/166157 A1 presents an orthodontic compliance monitor including a sensor that senses when an orthodontic appliance is properly positioned and a processor that processes an output of the sensor and generates compliance data. A memory device stores compliance data and the processor generates the compliance data based on a compliance protocol. System and methods for orthodontic compliance are also presented.

### DETAILED DESCRIPTION

An objective of the present invention is to provide a tooth-attach wearable device. The objective is reached by a tooth-attach wearable device as defined by the independent claim.

At least one example embodiment provides a tooth-attach wearable device that may verify whether a patient has worn a tooth-attach wearable device in an appropriate manner and may also verify an amount of time in which the patient has worn a transparent orthodontic device.

At least one example embodiment also provides a tooth-attach wearable device that may sense and record biometric information from the saliva of a patient, may store the sensed biometric information as data, and may remotely transmit the stored data.

A tooth-attach wearable device according to an aspect of at least one example embodiment includes a sensor device configured to sense biometric information of a patient, and a communication controller, comprising a data storage, configured to store the biometric information, including temperature information, sensed at the sensor device as data and to transmit the stored data.

Also, the sensor device includes a sensor head configured to contact with a tongue or gums of the patient or be exposed inside the oral cavity, and to sense the biometric information of the patient by sensing the saliva or a temperature of the patient, and a sensor chip configured to couple with the sensor head.

Also, a thermoelement is, and, optionally, at least one of a piezoelectric element, a chemical substance detection element, and a marker sensor may be, provided to the sensor head.

Also, the communication controller may include the data storage configured to store the biometric information of the patient sensed at the sensor device as data, and a data transmitter configured to remotely transmit the data stored in the data storage.

Also, the tooth-attach wearable device may further include a monitoring device configured to display the data transmitted from the data transmitter.

Also, the monitoring device may be provided to at least one of a desktop personal computer (PC), a laptop computer, a smartphone, and a display device for remote transmission in order to display the data.

Also, a portion of the sensor device, including a portion of the thermoelement, may be coated with a hydrophobic coating film to prevent a deformation by the saliva.

Also, a tooth-attach wearable device having a tooth attachment corresponding to a shape of a tooth according to at least one example embodiment includes a sensor head configured to couple with one surface of the tooth attachment, to be exposed inside the oral cavity, and to sense a temperature of a patient, and a time measurer configured to measure an amount of time in which the tooth attachment is attached to the tooth, based on the temperature sensed at the sensor head.

Also, the thermoelement is provided to the sensor head, the thermoelement may generate electricity at a preset temperature, and the electricity generated at the thermoelement may be supplied to the time measurer.

Also, the tooth-attach wearable device may further include a sensor chip configured to couple with the sensor head, to fix to the tooth attachment, and to supply electricity to the sensor head. A wirelessly chargeable battery may be provided to the sensor chip.

Also, the tooth-attach wearable device may further include a communication controller provided to the tooth attachment, and configured to store temperature information of the patient sensed at the sensor head, and to transmit the stored temperature information. The communication controller may include a data storage configured to store the temperature information of the patient sensed at the sensor head as data, and a data transmitter configured to remotely transmit the data stored in the data storage.

Also, the tooth-attach wearable device may further include a monitoring device configured to display the data transmitted from the data transmitter.

Also, the monitoring device may be provided to at least one of a desktop PC, a laptop computer, a smartphone, and a display device for remote transmission in order to display the data.

A tooth-attach wearable device having a tooth attachment corresponding to a shape of a tooth according to an aspect of at least one example embodiment includes a sensor head configured to couple with one surface of the tooth attachment, to be exposed inside the oral cavity, and to sense biometric information of a patient through contact between at least a portion of the exposed sensor head and the saliva of the patient, and a controller communication configured to fix to the tooth attachment, to store saliva information of the patient sensed at the sensor head, and to transmit the stored saliva information.

Also, a chemical substance detection element may be provided to the sensor head, and the chemical substance detection element may measure a blood sugar of the patient or may measure at least one of an amount, a type, and a change of hormone by sensing glucose in the saliva.

Also, a chemical substance detection element may be provided to the sensor head, and the chemical substance detection element may measure a bad breadth inside the oral cavity.

Also, the tooth-attach wearable device may further include a sensor chip configured to couple with the sensor head, to fix to the tooth attachment, and to supply electricity to the sensor head. A wirelessly chargeable battery may be provided to the sensor chip and electricity stored in the battery may be supplied to the sensor head.

Also, the sensor head may be configured to contact with at least one of a tooth, gums, and skin inside the oral cavity, and to measure at least one of a blood pressure, an electrocardiogram (ECG), and a pulse.

Also, the tooth-attach wearable device may further include a monitoring device configured to display data transmitted from the data transmitter.

Also, the tooth-attach wearable device may further include at least one saliva container formed on the tooth attachment, and each saliva container provided around the sensor head in a shape of a bowl capable of containing the saliva of the patient.

A tooth-attach wearable device having a tooth attachment corresponding to a shape of a tooth according to another aspect of at least one example embodiment, includes the sensor head configured to couple with one surface of the tooth attachment, and to sense an engagement force between the tooth and the tooth attachment, and the communication controller configured to fix to the tooth attachment, to store magnitude information of the engagement force sensed at the sensor head, and to transmit the stored magnitude information.

Also, a piezoelectric element may be provided to the sensor head, and the piezoelectric element may sense an engagement force between a top surface of the tooth and the tooth attachment, and may record the presence or absence of bruxism and a bite force of the patient.

Also, the tooth-attach wearable device may further include a sensor chip configured to couple with the sensor head, to fix to the tooth attachment, and to supply electricity to the sensor head. A wirelessly chargeable battery may be provided to the sensor chip and electricity stored in the battery is supplied to the sensor head.

Also, an electric energy may be generated in response to a pressure applied to the piezoelectric element, and a battery configured to store the electric energy generated at the piezoelectric element may be provided.

Also, the communication controller may be provided on a side of the tooth attachment.

Also, the tooth-attach wearable device may further include a monitoring device configured to display data transmitted from the data transmitter.

Also, the monitoring device may be provided to at least one of a desktop PC, a laptop computer, a smartphone, and a display device for remote transmission in order to display the data.

A tooth-attach wearable device having a tooth attachment corresponding to a shape of a tooth according to another aspect of at least one example embodiment includes a touch sensor provided on one surface of the tooth attachment, and provided with at least one touch panel touchable by a tongue, and a transmitter configured to fix to the tooth attachment, and to transmit a signal in response to pushing the touch sensor.

Also, the transmitter may be configured to transmit a signal to a computer, and a cursor of the computer may be configured to move in response to a motion of the tongue contacted on the touch sensor.

A tooth-fix sensing device to fix on the surface of a tooth according to another aspect of at least one example embodiment includes an orthodontic bracket configured to attach to at least a portion of the tooth, a sensor head provided to the orthodontic bracket, and configured to be exposed inside the oral cavity, and to sense biometric information through contact with the saliva of a patient, a sensor chip configured to couple with the sensor head, and provided to the orthodontic bracket to supply electricity to the sensor head, and a communication controller configured to couple with the sensor chip, and provided to the orthodontic bracket to store biometric information of the patient sensed at the sensor head and to transmit the stored biometric information of the patient.

A tooth-fix sensing device to fix to an alveolar bone or gums according to another aspect of at least one example embodiment includes a mini screw configured to implant in at least one of the alveolar bone and the gums, a sensor head provided to the mini screw, and configured to be exposed inside the oral cavity and to sense biometric information of a patient in contact with the saliva of the patient, a sensor chip configured to couple with the sensor head, to fix to the mini screw, and to supply electricity to the sensor head, and a communication controller configured to couple with the sensor chip, to fix to the mini screw, to store the biometric information of the patient sensed at the sensor head, and to transmit the stored biometric information of the patient.

Also, the mini screw may include a mini screw head configured to protrude from the periodontal tissue, to couple with the sensor head, and to sense the saliva of the patient, and a mini screw body configured to detachably couple with the mini screw head, to insert into the periodontal tissue, and to fix to the alveolar bone.

A tooth-attach wearable device attachable to a portion of a tooth and having a tooth attachment corresponding a shape of the tooth according to another aspect of at least one example embodiment includes a sensor head configured to couple with one surface of the tooth attachment, to be exposed inside the oral cavity, and to sense the saliva or a temperature of a patient, a sensor chip configured to couple with the sensor head, to fix to the tooth attachment, and to supply electricity to the sensor head, and a communication controller configured to couple with the sensor chip, to fix to the tooth attachment, to store saliva information or temperature information of the patient sensed at the sensor head, and to transmit the stored saliva information or temperature information of the patient.

Also, a cutting line may be formed on a portion of the middle of the tooth attachment to expose the tooth.

A tooth-attach wearable device having a tooth attachment corresponding to a shape of a tooth according to another aspect of at least one example embodiment includes a snoring sensor configured to couple with one surface of the tooth attachment, and to sense at least one of a vibration, an oxygen saturation, and sound by snoring, a sensor chip configured to couple with the vibration sensor, to fix to the tooth attachment, and to supply electricity to the vibration sensor, and a communication controller configured to couple with the sensor chip, to fix to the tooth attachment, to store vibration information sensed at the vibration sensor, and to store the stored vibration information.

Also, the communication controller may include a data storage configured to store the vibration information sensed at the snoring sensor as data, and a data transmitter configured to remotely transmit the data stored in the data storage.

Also, the tooth-attach wearable device may further include a monitoring device configured to display the data transmitted from the data transmitter.

Also, the snoring sensor may configured to sense at least one of a vibration occurring between a nose and vocal chords, an oxygen saturation occurring in breathing, and a snoring sound.

According to example embodiments, if a patient wears a transparent orthodontic device, it is possible to measure an amount of time in which the patient is wearing the transparent orthodontic device. Accordingly, the patient may be enabled to voluntarily wear the transparent orthodontic device for a desired period of time or more.

Also, according to example embodiments, a therapist, such as dentist, may verify an orthodontic state of a patient in real time and may perform various examinations based on biometric information.

Also, according to example embodiments, it is possible to verify information transferred to a tooth-attach wearable device using a portable device, such as a smartphone, without restrictions on a time and an occasion.

### BRIEF DESCRIPTION OF DRAWINGS

FIG. 1 is a perspective view illustrating a tooth-attach wearable device according to a first example embodiment.
FIG. 2 is block diagram illustrating a tooth-attach wearable device according to the first example embodiment.
FIG. 3 is a view illustrating a thermoelement provided to a sensor head of a lower tooth-attach wearable device according to the first example embodiment.
FIG. 4 is a view illustrating a chemical substance detection element provided to a sensor head of a lower tooth-attach wearable device according to a second example embodiment.
FIG. 5 is a view illustrating a piezoelectric element provided to a sensor head of a lower tooth-attach wearable device according to a third example embodiment.
FIG. 6 is a block diagram illustrating a vibration sensor provided to a tooth-attach wearable device according to a fourth example embodiment.
FIG. 7 is a view illustrating a touch sensor provided to a lower tooth-attach wearable device according to a fifth example embodiment.
FIG. 8 is a view illustrating a tooth-attach wearable device attached to a portion of teeth of a patient according to a sixth example embodiment.
FIG. 9 is a view illustrating a tooth-fix sensing device according to a seventh example embodiment.
FIG. 10 is a view illustrating a tooth-fix sensing device observed from side according to the seventh example embodiment.
FIG. 11 is a view illustrating a tooth-fix sensing device according to an eighth example embodiment.
FIG. 12 illustrates an example of remotely transferring biometric information of a patient using a communication controller of a tooth-attach wearable device according to the first example embodiment.

### DETAILED DESCRIPTION TO CARRY OUT THE DISCLOSURE

Hereinafter, example embodiments will be described with reference to the accompanying drawings.

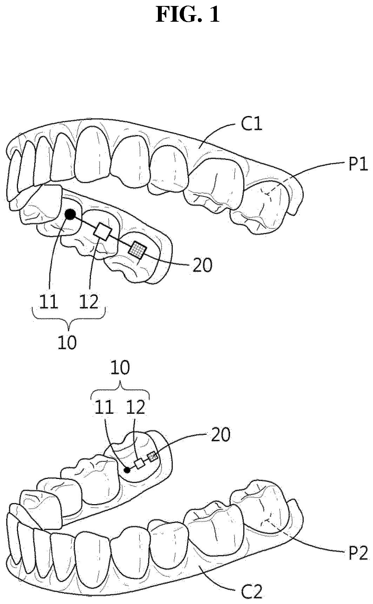

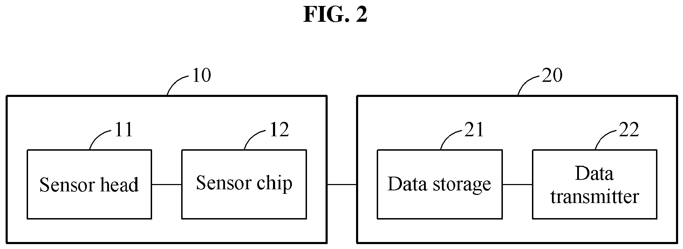

FIG. 1 is a perspective view illustrating a tooth-attach wearable device according to a first example embodiment, and FIG. 2 is a block diagram illustrating a tooth-attach wearable device according to the first example embodiment.

Hereinafter, a description will be made based on an example in which a tooth-attach wearable device C1, C2 according to an example embodiment is a transparent orthodontic device. Although the description is described herein based on the example in which the tooth-attach wearable device C1, C2 is a transparent orthodontic device, the present disclosure is not limited to a transparent material or an orthodontic device. Thus, any type of devices detachably attached to teeth may be applicable.

The transparent orthodontic device may be formed using polyethylene terephthalate (PET) or a specially reinforced plastic material. The specially reinforced plastic material has some advantages, such as a relatively great strength, a significant lightness, and a small abrasion compared to a general plastic. Thus, the specially reinforced plastic material does not add difficulty to teeth and is not easily deformed even after long use. In addition, if the specially reinforced plastic material is formed using a transparent specially reinforced plastic, an aesthetic property is excellent in that a patient may not appear to wear the orthodontic device although the patient wears the orthodontic device.

Referring to FIGS. 1 and 2, the tooth-attach wearable device C1, C2 may include a sensor device 10 and a communication controller 20. In more detail, the tooth-attach wearable device C1, C2 may be divided into an upper tooth-attach wearable device C1 and a lower tooth-attach wearable device C2.

The sensor device 10 refers to a portion that becomes into contact with a tongue or gums of the patient, and may be exposed outside a tooth attachment P1, P2 formed on the tooth-attach wearable device C1, C2.

The sensor device 10 may include a sensor head 11 configured to sense biometric information of the patient by sensing the saliva or a temperature of the patient, and a sensor chip 12 configured to couple with the sensor head 11. One of a thermoelement 11a, a piezoelectric element 11b, a chemical substance detection element 11c, and a marker sensor (not shown) may be provided to the sensor head 11. However, the example embodiments are not limited thereto.

In particular, the sensor head 11 included in the sensor device 10 is a portion that is exposed outside the tooth attachment P1, P2. Thus, a portion of the sensor head 11 may be coated with a hydrophobic coating film not be deformed by the saliva of the patient, etc. On the contrary, the sensor chip 12 and the communication controller 20, which will be described below, are embedded in the tooth attachment P1, P2 and thereby coated and thus, may be completely prevented from the saliva.

The communication controller 20 may include a data storage 21 configured to store the biometric information sensed at the sensor device 10 as data, and a data transmitter 22 configured to remotely transmit the data stored in the data storage 21. Here, a monitoring device M, M' configured to display the data transmitted from the data transmitter 22 may be further included. For example, the monitoring device M, M' may refer to a desktop personal computer (PC), a laptop computer, a smartphone, and other various types of display devices capable of performing remote transmission. The above various types of display devices capable of performing remote transmission may be any type of objects on which images, letters, numbers, etc., can be displayed. For example, a window, a front glass window of a vehicle, etc., may be included in the various types of display devices capable of performing remote transmission.

The communication controller 20 may couple with the tooth-attach wearable device C1, C2 by being internally molded therein.

Meanwhile, referring to FIG. 1, the sensor device 10 and the communication controller 20 may be mounted across a plurality of teeth and may be mounted to a single tooth.

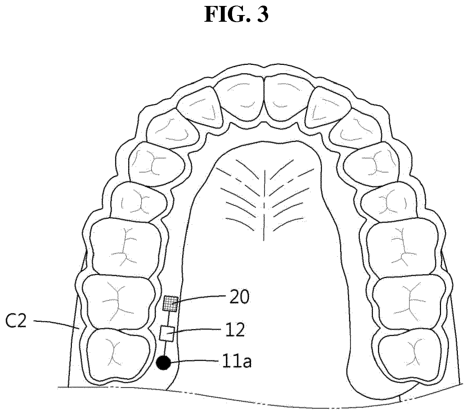

FIG. 3 is a view illustrating a thermoelement provided to a sensor head of a lower tooth-attach wearable device according to the first example embodiment.

Referring to FIG. 3, the thermoelement 11a may be provided to the sensor head 11 of the lower tooth-attach wearable device C2 according to the example embodiment.

In detail, the sensor device 10 may sense whether the tooth-attach wearable device C1, C2 is attached to teeth by sensing a temperature of the patient. Once the tooth-attach wearable device C1, C2 is sensed to be attached, the sensor device 10 may measure an amount of time in which the tooth-attach wearable device C1, C2 is attached to the teeth. If the tooth-attach wearable device C1, C2 is a transparent orthodontic device, the sensor device 10 or the communication controller 20 may include a time measurer (not shown) configured to record an amount of time from a time at which the tooth-attach wearable device C1, C2 is attached to the teeth to a time at which the tooth-attach wearable device C1, C2 is detached (separate) from the teeth. The time measurer may be set to measure and accumulate an amount of time only in response to receiving electricity being supplied from the thermoelement 11a. The thermoelement 11a may be set to generate electricity and to supply the electricity to the time measurer only when the temperature measured at the sensor device 10 is between about 35°C and about 38°C, which is close to a general human temperature. A measured temperature range such that the thermoelement 11a generates the electricity may be appropriately modified based on a situation of a user, for example, the patient.

In the above manner, an amount of time in which the transparent orthodontic device is attached to the teeth may be measured. Since the patient is allowed to freely put on or take out the transparent orthodontic device, it is important for the patient to voluntarily wear the orthodontic device in order to enhance the orthodontic effect. The transparent orthodontic device may be applicable to any age range except for a child of which physique basically varies. However, when the patient voluntarily wears the orthodontic device for 17 hours or more, the orthodontic effect may be acquired. For example, if the average amount of time in which the patient wears the orthodontic device is measured at the time measurer to be less than 17 hours a day, it may be determined that the orthodontic treatment period may increase.

Accordingly, the sensor device 10 may sense that the patient puts on, that is, attaches the tooth-attach wearable device C1, C2 to teeth, and may provide information used to determine the orthodontic period and the orthodontic state of the patient.

The thermoelement 11a is exposed outside the tooth attachment P2 and may directly contact with the saliva of the patient. Here, a portion of the sensor device 10 may be coated with a hydrophobic coating film (not shown) to prevent deformation or malfunction by the saliva. The hydrophobic coating film directly contacts with a tooth and thus, may be formed using a material that may not damage the tooth.

A wirelessly chargeable battery (not shown) may be provided to the sensor chip 12, and electricity stored in the battery may be supplied to the sensor head 11. The sensor chip 12 may be wirelessly charged from the wireless charging device in a form of an ear ring, a headset, or Bluetooth that couples with an ear in a state in which the tooth-attach wearable device C1, C2 is attached to the teeth of the patient. Alternatively, if the tooth-attach wearable device C1, C2 is not used, the battery of the sensor chip 12 may be charged by placing the tooth-attach wearable device C1, C2 on the wireless charging device.

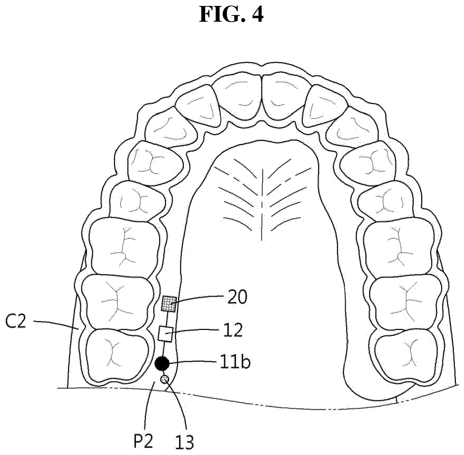

FIG. 4 is a view illustrating a chemical substance detection element provided to a sensor head of a lower tooth-attach wearable device according to a second example embodiment.

Referring to FIG. 4, the lower tooth-attach wearable device C2 according to the second example embodiment may include the sensor head 11, the sensor chip 12, a saliva container 13, and the communication controller 20. Here, the chemical substance detection element 11b may be provided to the sensor head 11.

The sensor head 11 may couple with one surface of the tooth attachment P2, and may be exposed inside the oral cavity, and may sense the saliva of the patient. In more detail, the chemical substance detection element 11b may sense glucose among a plurality of components included in the saliva and may measure blood sugar of the patient. In addition, the chemical substance detection element 11b may measure amounts or types of various hormones, such as a cortisol hormone, a change therein, and the like, and may detect the presence or absence of various types of diseases.

Acquiring biometric information of the patient from the saliva of the patient may be a new method capable of replacing a standard blood test. The saliva generally includes about 99 % of moisture, whereas important biometric information may be acquired from remaining 1% of the saliva. In addition to biological information and genetic information, materials helpful to fight against the diseases may be acquired from the saliva. In particular, various types of biometric information may be easily acquired using the saliva. For example, a specific protein used as a cardiac risk index in a blood test may be acquired from the saliva. Thus, such a saliva test may replace the blood test.

The saliva may be positioned in a lower portion of the oral cavity due to gravity. Thus, the sensor head 11 for sensing the saliva may be provided to the lower tooth-attach wearable device C2.

The saliva sensed at the sensor head 11 may be spit and may also be a component, such as a gingival crevicular fluid secreted between teeth and gums. The gingival crevicular fluid refers to the saliva discharged from above a boundary between the teeth and the gingiva, and may have a relatively high medical accuracy in the aforementioned hormone related measurement compared to the spit.

Meanwhile, the chemical substance detection element 11b may measure bad breadth inside the oral cavity. The chemical substance detection element 11b may employ any known configuration to measure the bad breadth. The chemical substance detection element 11b for measuring the bead breadth may measure a level of bad breadth based on the saliva inside the oral cavity or the air inside the oral cavity.

The chemical substance detection element 11b may be positioned at a location corresponding to an innermost tooth attachment P2 of the lower tooth-attach wearable device C2, that is, the tooth attachment P2 in contact with an innermost molar since the parotid gland and the submandibular gland are largest among salivary glands of a human being and the parotid gland and the submandibular gland are positioned to be closest to molars among teeth. The stress may increase adrenaline and the secretion of the saliva, and may make a heart beat quickly. Here, an enzyme, such as alpha-amylase, is secreted in the salivary gland. A stress diagnosis may be performed through cortisol hormone measurement, and may be used as an index of stress.

The sensor chip 12 may couple with the sensor head 11, and may be fixed to the lower tooth attachment P2 and supply the electricity to the sensor head 11. Here, a wirelessly chargeable battery may be provided to the sensor chip 12, and electricity stored in the battery may be supplied to the sensor head 11. The sensor chip 12 may be wirelessly charged from the wireless charging device in a form of an ear ring, a headset, or Bluetooth that couples with an ear in a state in which the lower tooth-attach wearable device C2 is attached to the teeth of the user. Alternatively, if the lower tooth-attach wearable device C2 is not used, the battery of the sensor chip 12 may be charged by placing the lower tooth-attach wearable device C2 on the wireless charging device.

At least one saliva container 13 may be formed on the tooth attachment P2, and may be formed in a shape, such as a bowl, capable of containing the saliva of the patient. The saliva container 13 may be provided at a location adjacent to the sensor head 11 so that the sensor head 11 may sense the saliva of the patient contained in the saliva container 13.

In addition to the blood sugar, the sensor head 11 may also measure biometric information, such as blood pressure, electrocardiogram (ECG), and pulse. The sensor head 11 for measuring the blood pressure or ECG may not be exposed from the tooth attachment P1, P2. In detail, the sensor head 11 for measuring the blood pressure or the ECG may not be exposed from the tooth attachment P1, P2 toward an inside of the oral cavity, and may measure the blood pressure or the ECG in contact with the gums, etc. A known blood pressure or ECG measurement device may be provided to the sensor head 11 and may measure the blood pressure or the ECG. The sensor head 11 for measuring biometric information, such as blood pressure or ECG may not be necessarily provided to the lower tooth-attach wearable device C2, and may be provided to the upper tooth-attach wearable device C1.

Meanwhile, a marker sensor capable of sensing a bio-marker and the like may be provided to the sensor head 11. The marker sensor may verify various types of hormone indices by verifying a specific genetic trait included in the saliva and may diagnose various types of diseases accordingly.

The communication controller 20 may include the data storage 21 configured to store the saliva information sensed at the sensor head 11 as data and the data transmitter 22 configured to remotely transmit the data stored in the data storage 21. Here, the monitoring device M, M' configured to display the data transmitted from the data transmitter 22 may be further included. For example, the monitoring device M, M' may be a desktop PC, a laptop computer, a smartphone, and any type of display devices capable of performing remote transmission.

The communication controller 20 may couple with the tooth-attach wearable device C1, C2 by being internally molded therein.

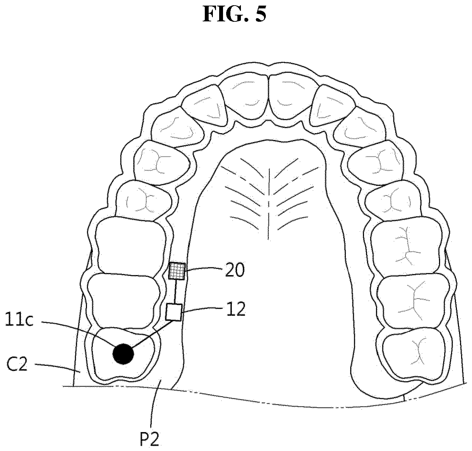

FIG. 5 is a view illustrating a piezoelectric element provided to a sensor head of a lower tooth-attach wearable device according to a third example embodiment.

Referring to FIG. 5, the lower tooth-attach wearable device C2 according to the third example embodiment may include the sensor head 11, the sensor chip 12, and the communication controller 20. The piezoelectric element 11c may be provided to the sensor head 11.

The sensor head 11 may couple with one surface of the tooth attachment P2, that is, an engagement surface, and may sense an engagement force between a tooth and the tooth attachment P2. Here, the sensor head 11 may be provided on the engagement surface of the tooth attachment P2 that faces a top surface of the tooth of the patient. If the patient puts on the lower tooth-attach wearable device C2 at the lower teeth, the sensor head 11 may sense a force occurring when the lower teeth and the upper teeth are engaged in response to shutting the patient's mouth. Accordingly, the sensor head 11 may sense the engagement force between the tooth and the tooth attachment P2, may determine whether bruxism of the patient is present, may measure the engagement force between the upper teeth and the lower teeth, and may verify the effect of measured engagement force against the teeth.

In detail, since an amount of electricity generated at the piezoelectric element 11c increases according to an increase in pressure applied to the sensor head 11, the piezoelectric element 11c provided to the sensor head 11 may determine whether bruxism of the patient is present and may measure the engagement force between the upper teeth and the lower teeth by measuring the electricity generated at the piezoelectric element 11c.

Also, since the engagement force occurring when the patient shuts the patient's mouth is sensed at the sensor head 11, the sensor chip 12 and the communication controller 20 may be provided to the side of the tooth attachment P2 instead of being provided to the top surface of the tooth attachment P2.

Once the piezoelectric element 11c is provided to the sensor head 11, it is possible to determine a temporomandibular disorder of the patient. The temporomandibular disorder may be solved by employing an in-mouth device in a form of orthodontic treatment or mouthpiece. The mouthpiece may be applicable to treat the temporomandibular disorder by applying principles of the tooth-attach wearable device C1, C2 to the mouthpiece.

The sensor chip 12 may couple with the sensor head 11, and may be fixed to the lower tooth attachment P2 and supply electricity to the sensor head 11. Here, a wirelessly chargeable battery may be provided to the sensor chip 12, and electricity stored in the battery may be supplied to the sensor head 11. The sensor chip 12 may be wirelessly charged from the wireless charging device in a form of an ear ring, a headset, or Bluetooth that couples with an ear in a state in which the lower tooth-attach wearable device C2 is attached to the teeth of the user. Alternatively, if the lower tooth-attach wearable device C2 is not used, the battery of the sensor chip 12 may be charged by placing the lower tooth-attach wearable device C2 on the wireless charging device.

Alternatively, electric energy generated at the piezoelectric element 11c with chewing pressure energy of the user may be stored in the battery. Such energy stored in the battery may be used to drive the sensor device 10 and the communication controller 20, etc. Accordingly, using the electric energy generated when a pressure is applied to the piezoelectric element 11c, the piezoelectric element 11c may determine whether bruxism of the patient is present and may measure the engagement force between the upper teeth and the lower teeth. Alternatively, the piezoelectric element 11c may store the electric energy and may provide the electric energy to other constituent elements, such as the sensor device 10 and the communication controller 20, etc.

The communication controller 20 may include the data storage 21 configured to store magnitude information of the engagement force between the tooth and the tooth attachment P2 sensed at the sensor head 11 as data and the data transmitter 22 configured to remotely transmit the data stored in the data storage 21. Here, the monitoring device M, M' configured to display the data transmitted from the data transmitter 22 may be further included. For example, the monitoring device M, M' may be a desktop PC, a laptop computer, a smartphone, and any type of display devices capable of performing remote transmission

The communication controller 20 may couple with the tooth-attach wearable device C1, C2 by being internally molded therein.

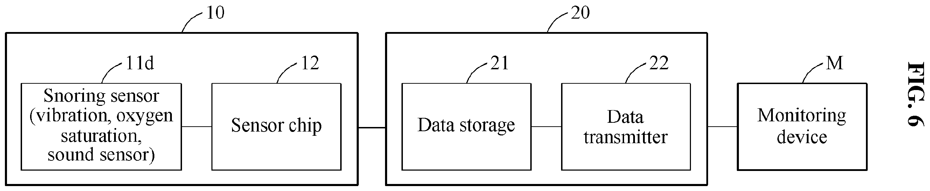

FIG. 6 is a block diagram illustrating a vibration sensor provided to a tooth-attach wearable device according to a fourth example embodiment.

Referring to FIG. 6, the tooth-attach wearable device C1, C2 according to the fourth example embodiment may include a vibration sensor 11d, the sensor chip 12, the communication controller 20, and the monitoring device M.

The vibration sensor 11d may couple with one surface of the tooth attachment P1, P2 and may sense a vibration by snoring. In more detail, the vibration sensor 11d may sense a vibration by snoring occurring between a nose and vocal chords. In general, air passages may become narrow and air may flow when the user breathes. In this instance, snoring may occur while causing friction, a vibration, etc., in uvula or plate of the user. Accordingly, the vibration sensor 11d may sense a vibration occurring when the air flows through air passages and may sense the occurrence of snoring during a sleep.

The sensor chip 12 may couple with the vibration sensor 11d, and may be fixed to the tooth attachment P1, P2 and supply electricity to the vibration sensor 11d.

The communication controller 20 may couple with the sensor chip 12, and may be fixed to the tooth attachment P1, P2.

The communication controller 20 may store vibration information sensed at the vibration sensor 11d and may serve to transmit the stored vibration information.

The communication controller 20 may include the data storage 21 configured to store the vibration information sensed at the vibration sensor 11d as data and the data transmitter 22 configured to remotely transmit the data stored in the data storage 21. Here, the monitoring device M configured to display the data transmitted from the data transmitter 22 may be included.

That is, the vibration sensor 11d may determine the presence or absence of snoring by sensing a vibration by snoring occurring between the nose and the vocal chords, and by displaying the transmitted vibration information on the monitoring device M.

Also, in addition to the method of sensing a vibration by snoring, it is possible to verify an oxygen saturation occurring when the patient breathes or a sound occurring when the patient snores. In the case of sensing the oxygen saturation, an oxygen saturation sensor may be used instead of the vibration sensor. In the case of sensing the sound, a sound sensor may be used instead of the vibration sensor.

If the oxygen saturation is less than an average value, the oxygen saturation may be used as an index to determine sleep disorders, such as snoring, obstructive sleep apnea, etc., occurring due to lack of oxygen, and diseases, such as a stroke, heart attack, etc. The saturation of oxygen sensed inside the oral cavity may decrease when the user snores compared to a case in which the user does not snore. Thus, it is possible to determine the presence or absence of snoring and to monitor a level of snoring in real time by sensing the oxygen saturation.

Also, when the user snores, a snoring sound occurs. Thus, it is possible to monitor the occurrence of sound and a change in a magnitude of sound in real time. The oxygen saturation and sound information sensed at the oxygen saturation sensor and the sound sensor may be transmitted to the monitoring device M, and may be used to determine the presence or absence of snoring and a level of snoring.

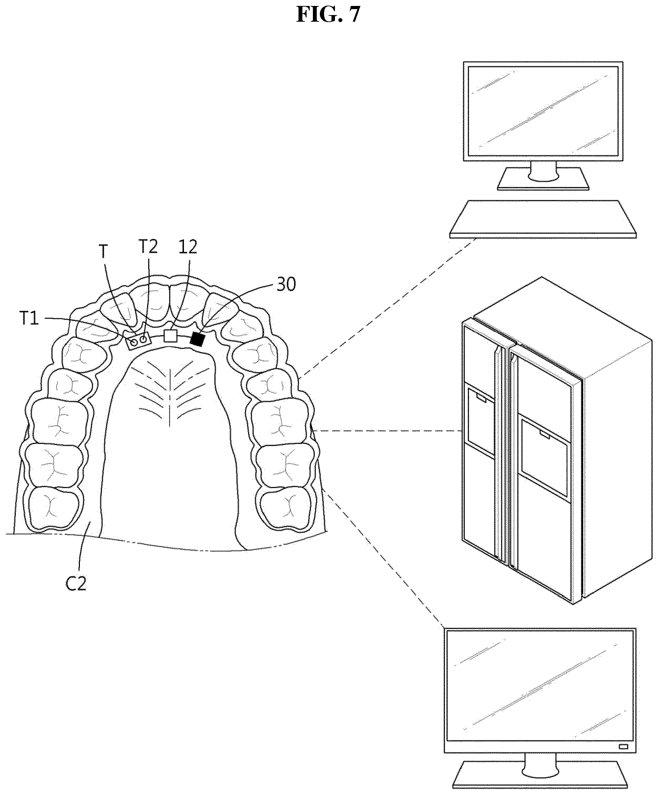

FIG. 7 is a view illustrating a touch sensor provided to a lower tooth-attach wearable device according to a fifth example embodiment.

Referring to FIG. 7, the lower tooth-attach wearable device C2 according to the fifth example embodiment may include a touch sensor T, the sensor chip 12, and a transmitter 30.

The touch sensor T may couple with one surface of the tooth attachment P2, and at least one touch panel T1, T2 may be provided.

For example, the touch sensor T may be provided in a structure similar to a mouth that includes at least two touch panels T1 and T2. In response to pushing one of the two touch panels T1 and T2, the transmitter 30 may transmit a signal to a computer, a TV, a refrigerator, a washing machine, a boiler, etc.

The sensor chip 12 may couple with the touch sensor T, and may be fixed to the tooth attachment P2 and supply electricity to the touch sensor T.

The transmitter 30 may couple with the sensor chip 12, may be fixed to the tooth attachment P2, and may transmit a signal to a computer and the like in response to pushing the touch panel T1, T2.

For example, the touch sensor T may contact with the tongue of the user and thus, may be positioned in an inner side of the middle of the lower tooth-attach wearable device C2 as shown in FIG. 7.

The user wearing the lower tooth-attach wearable device C2 according to the example embodiment may click a cursor of a computer by pushing the touch panel T1, T2 with the tongue of the user, and may also move the cursor on a monitor. The user wearing the lower tooth-attach wearable device C2 may power on a TV, a refrigerator, a washing machine, a boiler, etc., by pushing the touch panel T1, T2 with the tongue, or may transfer a signal to such devices and may manipulate the devices. For example, by pushing the touch panel T1, T2 with the tongue, the user wearing the lower tooth-attach wearable device C2 may power on a TV or select a channel, may power on a refrigerator or adjust an inside temperature of the refrigerator, and may power on a boiler or adjust an operating temperature of the boiler.

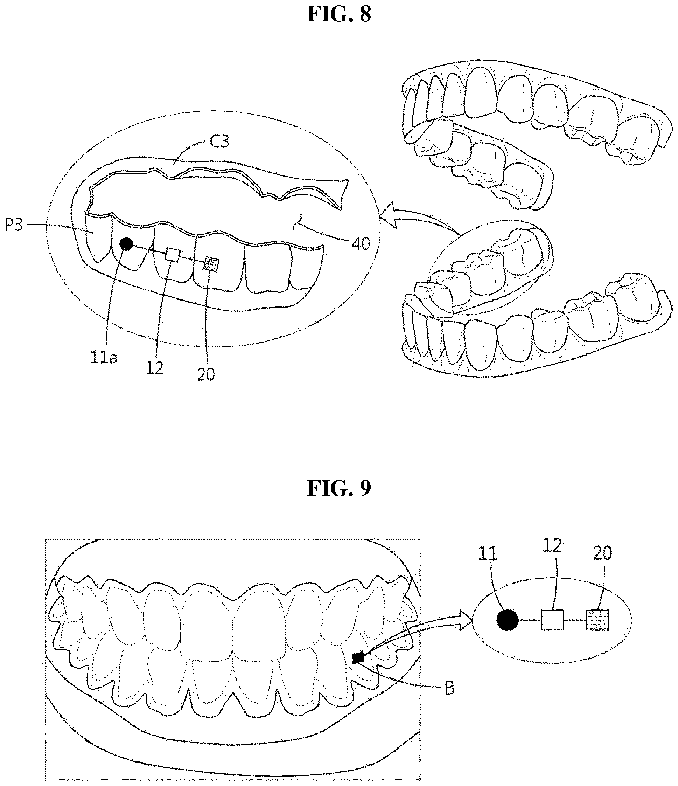

FIG. 8 is a view illustrating a tooth-attach wearable device attached to a portion of teeth of a patient according to a sixth example embodiment.

Referring to FIG. 8, a tooth-attach wearable device C3 according to the sixth example embodiment may include the sensor device 10 and the communication controller 20.

Also, a cutting line 40 of which one surface is cut may be formed on the tooth-attach wearable device according to the sixth example embodiment.

The thermoelement 11a or the chemical substance detection element 11b may be provided to the sensor head 11 of the sensor device 10. In this instance, the piezoelectric element 11c may not be provided to the sensor head 11 since an engagement force between a tooth and the tooth attachment P2 may not be measured due to the cutting line 40 formed on the tooth-attach wearable device.

The sensor head 11 may couple with one surface of a tooth attachment P3, and may be exposed inside the oral cavity and may sense the saliva or a temperature of the patient.

The sensor chip 12 may couple with the sensor head 11, and may be fixed to the tooth attachment P3 and supply electricity to the sensor head 11.

The communication controller 20 may couple with the sensor chip 12 and may be fixed to the tooth attachment P3. The communication controller 20 may store saliva information or temperature information sensed at the sensor head 11 and may transmit the stored saliva information or temperature information.

The tooth-attach wearable device C3 may be attached only to a desired tooth instead of being applied to all of the teeth. For example, referring to FIG. 8, the tooth-attach wearable device C3 may be attached to a molar portion. Without being limited to the size and the shape of FIG. 8, the tooth-attach wearable device C3 may be manufactured in various types.

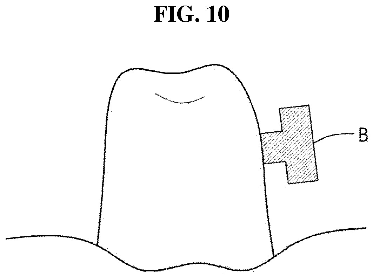

FIG. 9 is a view illustrating a tooth-fix sensing device according to a seventh example embodiment, and FIG. 10 is a view illustrating a tooth-fix sensing device observed from side according to the seventh example embodiment.

Referring to FIGS. 9 and 10, the tooth-fix sensing device according to the seventh example embodiment may be a plastic orthodontic bracket B fixed to a tooth (teeth).

The sensor head 11, the sensor chip 12, and the communication controller 20, etc., provided to the aforementioned tooth-attach wearable device C1, C2, C3 may also be applied to the plastic orthodontic bracket B in the same manner.

Here, since the plastic orthodontic bracket B is not detachably attached like the transparent orthodontic device, there is no need to sense a temperature of the user and to measure an amount of time in which the plastic orthodontic bracket B is attached to the teeth.

The tooth-fix sensing device may include the orthodontic bracket B provided to at least a portion of teeth, the sensor head 11 provided to the orthodontic bracket B and exposed inside the oral cavity to sense biometric information in contact with the saliva of the patient, the sensor chip 12 configured to couple with the sensor head 11 and provided to the orthodontic bracket B to supply electricity to the sensor head 11, and the communication controller 20 configured to couple with the sensor chip 12 and provided to the orthodontic bracket B to store the biometric information sensed at the sensor head 12 and to transmit the stored biometric information.

Here, a chemical substance detection element may be provided to the sensor head 11 that is provided to the orthodontic bracket B. The chemical substance detection element may sense glucose in the saliva and may measure blood sugar of the patient or may measure biometric information such as amounts or types of hormones, change therein, bad breathing, blood pressure, ECG, and pulse. Similar to other tooth-attach wearable devices C1, C2, C3, all of the sensor head 11, the sensor chip 12, and the communication controller 20 may be provided to a single plastic orthodontic bracket B.

The sensor head 11 may be exposed from the plastic orthodontic bracket B or may be embedded therein based on a type of an element provided to the sensor head 11. For example, in the case of sensing biometric information from the saliva of the patient, the sensor head 11 may be exposed inside the oral cavity, and the chemical substance detection element 11b may be provided to the sensor head 11.

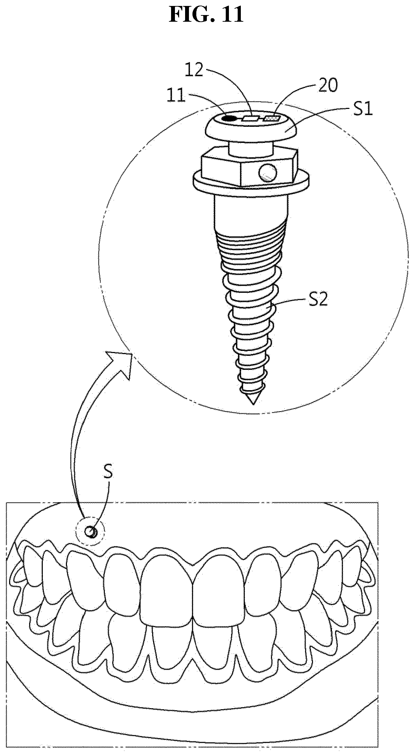

FIG. 11 is a view illustrating a tooth-fix sensing device according to an eighth example embodiment.

Referring to FIG. 11, the tooth-fix sensing device according to the eighth example embodiment may be a mini screw S that is connected to a plastic orthodontic bracket (not shown) using an elastic band and prevents a tooth being corrected from moving in an undesired direction.

The mini screw S may be fixed to the alveolar bone by passing through the gums, that is, periodontal tissue of the patient. The mini screw S may include a mini screw head S1 configured to be exposed outside the periodontal tissue and a mini screw body S2 configured to insert into the periodontal tissue. The mini screw head S1 may detachably couple with the mini screw body S2.

In more detail, the sensor head 11, the sensor chip 12, and the communication controller 20 may be provided to the mini screw head S1. The sensor head 11 may couple with the mini screw head S1, and may be exposed inside the oral cavity and sense the saliva. Accordingly, the sensor head 11 according to the eighth example embodiment may be provided as the chemical substance detection element 11b.

Also, although FIG. 11 illustrates that the sensor chip 12 and the communication controller 20 are included in the mini screw head S1 and are exposed inside the oral cavity, they except for the sensor head 11 for sensing biometric information from the saliva of the patient may be provided with being embedded in the mini screw head S1

The chemical substance detection element may be provided to the sensor head 11 that is provided to the mini screw head S1. The chemical substance detection element may sense glucose in the saliva and may measure blood sugar of the patient or may measure biometric information, such as amounts or types of hormones, a change therein, bad breath, blood pressure, ECG, and pulse.

The sensor chip 12 may couple with the sensor head 11, and may be fixed to the mini screw S and supply electricity to the sensor head 11.

The communication controller 20 may couple with the sensor chip 12, and may be fixed to the mini screw S, and may store the biometric information sensed at the sensor head 11 and may transmit the stored biometric information.

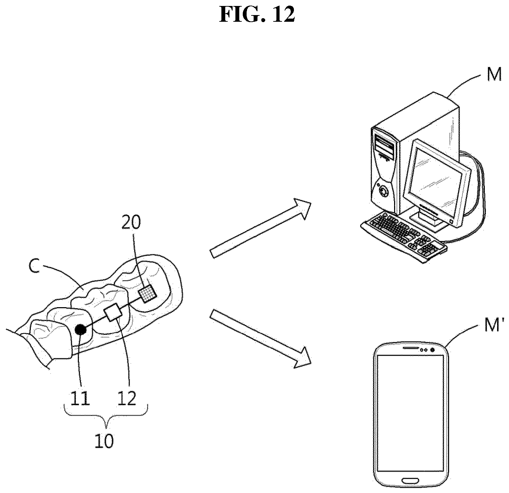

FIG. 12 illustrates an example of remotely transferring biometric information of a patient from a communication controller of a tooth-attach wearable device according to the first example embodiment.

Referring to FIG. 12, a tooth-attach wearable device C according to example embodiments may include the monitoring device M, M' configured to display data transmitted from the data transmitter 22.

The monitoring device M, M' may be, for example, a desktop PC M or a smartphone M'. Each of the desktop PC M and the smartphone M' may receive biometric information of the patient remotely transmitted from the data transmitter 22 provided to the tooth-attach wearable device C according to the example embodiments as well as the upper tooth-attach wearable device C1 and the lower tooth-attach wearable device C2.

It is possible to separately verify an amount of time in which the tooth-attach wearable device C is attached with respect to each of the upper teeth and the lower teeth using the monitoring device M, M'. Also, it is possible to analyze information obtained from the saliva and to use the analyzed information as various indices.

Hereinafter, an operation of the tooth-attach wearable device C according to the example embodiments will be described.

The tooth-attach wearable device C may include the sensor device 10 and the communication controller 20.

One of the thermoelement 11a, the chemical substance detection element 11b, and the piezoelectric element 11c may be provided to the sensor head 11 of the sensor device 10.

Initially, in an example in which the thermoelement 11a is provided to the sensor head 11, the sensor head 11 may sense a temperature of the patient at a location at which the sensor head 11 is exposed inside the oral cavity. For example, if the tooth-attach wearable device C is a transparent orthodontic device, the sensor head 11 may measure an amount of time in which the tooth-attach wearable device C is attached to the teeth by measuring the temperature of the patient, may check the measured amount of time in real time, and may use the checked amount of time to determine a subsequent orthodontic treatment direction. If the patient wears the transparent orthodontic device for less than a preset period of time, it is possible to inform the patient about an additional amount of time in which the patient is to wear the transparent orthodontic device.

In an example in which the chemical substance detection element 11b is provided to the sensor head 11, the sensor head 11 may sense the saliva of the patient at a location at which the sensor head 11 is exposed inside the oral cavity. In particular, the sensor head 11 may measure blood sugar of the patient by sensing glucose among components contained in the saliva of the patient.

In an example in which the piezoelectric element 11c is provided to the sensor head 11, the sensor head 11 may couple with a surface of the tooth attachment P1, P2 that faces a top surface of a tooth. Accordingly, the sensor head 11 may measure a magnitude of an engagement force between the tooth and the tooth attachment P1, P2 and may verify whether bruxism of the patient is present, etc.

The sensor chip 12 may couple with the sensor head 11. A wirelessly chargeable battery may be provided to the sensor chip 12, and may supply electricity stored in the battery to the sensor head 11.

The communication controller 20 may include the data storage 21 configured to store temperature information of the patient sensed at the sensor head 11 as data, and the data transmitter 22 configured to remotely transmit the data stored in the data storage 21. Here, the monitoring device M, M' configured to display the data transmitted from the data transmitter 22 may be further included.

Therefore, the tooth-attach wearable device C according to the example embodiments may monitor biometric information of the patient, and may use the biometric information as an index associated with an orthodontic state if the tooth-attach wearable device C is a transparent orthodontic device.

The tooth-attach wearable device C according to the example embodiment may be applicable to persons of various professions, such as an athlete, a soldier, etc.

## Claims

1. A tooth-attach wearable device (C, C1, C2, C3) having a tooth attachment (P1, P2, P3) corresponding to a shape of a tooth, the wearable device (C, C1, C2, C3) comprising:
a sensor head (11) configured to couple with one surface of the tooth attachment (P1, P2, P3), to be exposed inside the oral cavity, and to sense a temperature of a patient;
by wherein a time measurer configured to measure an amount of time in which the tooth attachment (P1, P2, P3) is attached to the tooth, based on the temperature sensed at the sensor head (11);
wherein a thermoelement (11a) is provided to the sensor head (11), the thermoelement (11a) generates electricity and supplies the generated electricity to the time measurer only when the temperature sensed at the sensor head (11) is between 35 °C and 38 °C;
wherein the time measurer measures and accumulates the amount of time only in response to receiving the electricity being supplied from the thermoelement (11a); and
wherein the wearable device (C, C1, C2, C3) further comprises a sensor chip (12) configured to couple with the sensor head (11), to fix to the tooth attachment (P1, P2, P3), and to supply electricity to the sensor head (11),
wherein the wearable device (C, C1, C2, C3) comprises a communication controller (20) provided to the tooth attachment (P1, P2, P3), and configured to store temperature information of the patient sensed at the sensor head (11), and to transmit the stored temperature information,
wherein the communication controller (20) comprises a data storage (21) configured to store the temperature information of the patient sensed at the sensor head (11) as data; and a data transmitter (22) configured to remotely transmit the data stored in the data storage (21),
wherein the thermoelement (11a) is exposed outside the tooth attachment (P1, P2, P3) and a portion of the thermoelement (11a) is coated with a hydrophobic film which is suitable for directly contacting with a tooth,
wherein the sensor head (11) is configured to sense an engagement force between the tooth and the tooth attachment (P1, P2, P3), and the communication controller (20) is configured to store magnitude information of the engagement force sensed at the sensor head (11), and the communication controller (20) transmits the stored magnitude information.

2. The wearable device (C, C1, C2, C3) of claim 1, wherein a piezoelectric element (11c) is provided to the sensor head (11), and the piezoelectric element (11c) senses an engagement force between a top surface of the tooth and the tooth attachment (P1, P2, P3), and records the presence or absence of bruxism and a bite force of the patient.

3. The wearable device (C, C1, C2, C3) of claim 2, wherein an electric energy is generated in response to a pressure applied to the piezoelectric element (11c), and a battery configured to store the electric energy generated at the piezoelectric element (11c) is provided.

## Patentansprüche

1. Am Zahn zu befestigendes tragbares Gerät (C, C1, C2, C3) mit einer Zahnbefestigung (P1, P2, P3) entsprechend einer Form eines Zahns, wobei das tragbares Gerät (C, C1, C2, C3) Folgendes umfasst:
einen Sensorkopf (11), der konfiguriert ist, um mit einer Oberfläche des Zahnaufsatzes (P1, P2, P3) gekoppelt zu werden, um innerhalb der Mundhöhle freigelegt zu werden, und um eine Temperatur eines Patienten zu erfassen;
durch einen Zeitmesser, der dazu konfiguriert ist, eine Zeitdauer zu messen, in der die Zahnbefestigung (P1, P2, P3) an dem Zahn befestigt ist, basierend auf der am Sensorkopf (11) erfassten Temperatur;
wobei am Sensorkopf (11) ein Thermoelement (11a) vorgesehen ist, das Thermoelement (11a) Strom erzeugt und den erzeugten Strom nur dann an den Zeitmesser liefert, wenn die am Sensorkopf (11) gemessene Temperatur zwischen 35 °C und 38 °C liegt;
wobei der Zeitmesser die Zeitdauer nur als Reaktion auf den Empfang der von dem Thermoelement (11a) gelieferten Elektrizität misst und akkumuliert; und
wobei das tragbare Gerät (C, C1, C2, C3) ferner einen Sensorchip (12) umfasst, der dazu konfiguriert ist, mit dem Sensorkopf (11) zu koppeln, um an dem Zahnaufsatz (P1, P2, P3) befestigt zu werden und den Sensorkopf (11) mit Strom zu versorgen,
wobei das tragbare Gerät (C, C1, C2, C3) eine Kommunikationssteuerung (20) umfasst, die an dem Zahnaufsatz (P1, P2, P3) bereitgestellt ist und dazu konfiguriert ist, am Sensorkopf (11) erfasste Temperaturinformationen des Patienten zu speichern und die gespeicherten Temperaturinformationen zu übertragen,
wobei die Kommunikationssteuerung (20) einen Datenspeicher (21), der konfiguriert ist, um die Temperaturinformationen des Patienten, die an dem Sensorkopf (11) erfasst werden, als Daten zu speichern; und einen Datensender (22) umfasst, der dazu konfiguriert ist, die im Datenspeicher (21) gespeicherten Daten aus der Ferne zu übertragen,
wobei das Thermoelement (11a) außerhalb des Zahnaufsatzes (P1, P2, P3) freiliegt und ein Teil des Thermoelements (11a) mit einem hydrophoben Film beschichtet ist, der zum direkten Kontakt mit einem Zahn geeignet ist,
wobei der Sensorkopf (11) konfiguriert ist, um eine Eingriffskraft zwischen dem Zahn und dem Zahnaufsatz (P1, P2, P3) zu erfassen, und die Kommunikationssteuerung (20) konfiguriert ist, um Größeninformationen der Eingriffskraft zu speichern, die an dem Sensorkopf (11) erfasst wird, und die Kommunikationssteuerung (20) die gespeicherten Größeninformationen überträgt.

2. Tragbares Gerät (C, C1, C2, C3) nach Anspruch 1, wobei am Sensorkopf (11) ein piezoelektrisches Element (11c) vorgesehen ist, und das piezoelektrische Element (11c) eine Eingriffskraft zwischen einer oberen Oberfläche des Zahns und der Zahnbefestigung (P1, P2, P3) erfasst und das Vorhandensein oder Fehlen von Bruxismus und eine Beißkraft des Patienten aufzeichnet.

3. Tragbares Gerät (C, C1, C2, C3) nach Anspruch 2, wobei eine elektrische Energie als Reaktion auf einen auf das piezoelektrische Element (11c) ausgeübten Druck erzeugt wird, und eine Batterie bereitgestellt wird, die konfiguriert ist, um die an dem piezoelektrischen Element (11c) erzeugte elektrische Energie zu speichern.

## Revendications

1. Dispositif portable de fixation de dent (C, C1, C2, C3) ayant une fixation de dent (P1, P2, P3) correspondant à la forme d'une dent, le dispositif portable (C, C1, C2, C3) comprenant :
une tête de capteur (11) conçue pour se coupler à une surface de la fixation de dent (P1, P2, P3), pour être exposée à l'intérieur de la cavité buccale et pour détecter une température d'un patient ;
dans lequel un appareil de mesure du temps conçu pour mesurer une durée pendant laquelle la fixation de dent (P1, P2, P3) est fixée à la dent, sur la base de la température détectée au niveau de la tête de capteur (11) ;
dans lequel un thermo-élément (11a) est fourni à la tête de capteur (11), le thermo-élément (11a) génère de l'électricité et fournit l'électricité générée à l'appareil de mesure du temps uniquement lorsque la température détectée au niveau de la tête de capteur (11) est comprise entre 35 °C et 38 °C ;
dans lequel l'appareil de mesure du temps mesure et accumule la quantité de temps uniquement en réponse à la réception de l'électricité fournie par le thermo-élément (11a) ; et
dans lequel le dispositif portable (C, C1, C2, C3) comprend en outre une puce de capteur (12) conçue pour se coupler à la tête de capteur (11), pour se fixer à la fixation de dent (P1, P2, P3) et pour fournir de l'électricité à la tête de capteur (11),
dans lequel le dispositif portable (C, C1, C2, C3) comprend un contrôleur de communication (20) fourni à la fixation de dent (P1, P2, P3), et conçu pour stocker des informations de température du patient détectées au niveau de la tête de capteur (11), et pour transmettre les informations de température stockées,
dans lequel le contrôleur de communication (20) comprend un stockage de données (21) conçu pour stocker les informations de température du patient détectées au niveau de la tête de capteur (11) en tant que données ; et un émetteur de données (22) conçu pour transmettre à distance les données stockées dans le stockage de données (21),
dans lequel le thermoélément (11a) est exposé à l'extérieur de la fixation de dent (P1, P2, P3) et une partie du thermoélément (11a) est revêtue d'un film hydrophobe qui convient pour entrer directement en contact avec une dent,
dans lequel la tête de capteur (11) est conçue pour détecter une force de prise entre la dent et la fixation de dent (P1, P2, P3), et le contrôleur de communication (20) est conçu pour stocker des informations d'amplitude de la force de prise détectée au niveau de la tête de capteur (11), et le contrôleur de communication (20) transmet les informations d'amplitude stockées.

2. Dispositif portable (C, C1, C2, C3) selon la revendication 1, dans lequel un élément piézoélectrique (11c) est fourni à la tête de capteur (11), et l'élément piézoélectrique (11c) détecte une force de prise entre une surface supérieure de la dent et la fixation de dent (P1, P2, P3), et enregistre la présence ou l'absence de bruxisme et une force de morsure du patient.

3. Dispositif portable (C, C1, C2, C3) selon la revendication 2, dans lequel une énergie électrique est générée en réponse à une pression appliquée à l'élément piézoélectrique (llc), et une batterie conçue pour stocker l'énergie électrique générée au niveau de l'élément piézoélectrique (11c) est fournie.
